# EUROPEAN PATENT APPLICATION

(11) **EP 4 461 141 A2**
(43) Date of publication of application: **13.11.2024**
(21) Application number: 24202801.7
(22) Date of filing: 08.11.2018
(51) Int. Cl.: A23K 20/158

(54) **POWDER COMPOSITION AND GEL COMPOSITION COMPRISING AQUATIC PHOTOSYNTHESIZING ORGANISMS**

(62) Divisional of application: 18000883.1
(71) Applicant: Biochem Zusatzstoffe Handels- und Produktionsges. mbH, 49393 Lohne (DE)
(72) Inventor: AYECKE-THUN, Marika, 49393 Lohne (DE); FRISCH, Melanie, 49393 Lohne (DE); REINERS, Christine, 26169 Friesoythe (DE)
(74) Representative: Aera A/S

(57) **Abstract**

The present invention relates to a powder composition for use in feeding animals, especially poultry comprising at least one gelling agent, at least one carrier and a prebiotic additive, wherein the prebiotic additive is an aquatic photosynthesizing organism. The present invention also relates to a gel composition comprising the powder composition and water as well as a method for administering the same.

## Description

The present invention relates to a powder composition for use in feeding animals and a gel composition for use in feeding animals comprising the powder composition. The invention further relates to a method for administering the gel composition as well as the use of the powder and the gel composition.

Providing animal feed with high nutritional value is of great importance especially for new born animals. In particular for non-mammals such as birds and in particular poultry it is important that the hatchlings are fed sufficiently.

In the poultry industry eggs are hatched in a hatchery and the hatchlings, also called chicks, are transported a few days after their hatch to a farm. For disease control and an increased health status, newly hatched poultry is vaccinated.

In recent times feed additives such as probiotics or vitamins are fed to the new born animals in particular chicks as early as in the hatchery in order to improve their health conditions and prevent diseases. To administer these additives, carrier substances are sometimes used. However, those compositions lack further benefits such as advantageous nutritional benefits, that is, providing substances to the newborns which increase their physical state as such.

The document WO 2009/033244 A1 refers to a nutritional formulation for post hatched birds comprising different vitamins, proteins and further additives such as dyes.

In the poultry industry known feeding substances, including but not limited to feed supplements and vaccines, for chicks are regularly administered in liquid form. They can be administered via the drinking water or they are sprayed on the animals. Spraying may lead to soaking of the feathers of the chicks causing a reduction of body temperature which is especially for newly hatched chicks critical as it may lead even to death.

In order to reduce the soaking of the feathers more solid compositions such as gels or foams have been proposed to be used as carrier substances. These applications have the advantage that they do not soak the feathers.

WO 03/045272 A2 relates to an oral, immunostimulating material for mammals, birds, fish and reptiles which comprises an immunostimulating amount of alginate and an acceptable carrier.

In EP 2 459 222 B1 a sticky soft gel for vaccinating a flock of poultry is disclosed. In this document the gel is used to deliver a vaccine agent to the poultry in a barn.

Such known compositions have the problem that they are difficult to be detected by the animals. However, colouring of feeding compositions cannot be easily performed as animal feeding is subject to various restrictions with respect to compatibility with the new born animals, visibility and durability of the colouring so that respective compositions can be stored. Besides, legal requirements in the area are strict as well.

With respect to the visibility, the disadvantage of the prior art is that the compositions often do not provide sufficient contrast so that the animals and in particular the hatchlings cannot see them properly. When not seen by the animals, in particular hatchlings, the intake rate is reduced.

Other known systems lack the long-lasting durability of the colouring agent so that they cannot be stored or shipped. Besides, premature degradation of such compounds may reduce the quality of the feed additive composition or even produce poisonous by-products. Another aspect is that various colouring agents are not allowed to be used for animal applications by some feed law regulations. Furthermore, some colouring agents have to be withdrawn from a vial with a needle and syringe, posing a risk of injury to the person applying it and may not be dosed accurately.

Hence, there is the need for feeding compositions providing improved nutritional value in particular for new born animals and also promoting the intake of the compositions in particular by new born animals such as chicks. Besides, the administration of the feeding compositions needs to be simple and effective. Another aspect is that it would be advantageous if such compositions would fulfil legal requirements, that is, comprise additives being approved by respective authorities.

It is therefore the object of the present invention to provide feeding compositions which have improved nutritional properties and which can be administered in a simple fashion. Besides, the intake of the feeding compositions by animals, in particular newborns such as chicks, should be improved as well.

This object is solved by the present invention. The invention is directed to a powder composition for use in feeding animals and a gel composition for use in feeding animals comprising the powder composition. The invention is also directed to a method for administering the gel composition as well as the use of the powder and the gel composition.

One aspect of the invention relates to a powder composition for use in feeding animals as defined by clause 1. Preferred embodiments of the powder composition are provided in the dependent claims and the description of the invention, which can optionally be combined with each other.

Another aspect of the invention relates to a gel composition for use in feeding animals as defined by clause 8. Preferred embodiments of the gel composition are provided in the dependent claims and the description of the invention, which can optionally be combined with each other.

A further aspect of the invention relates to a method for administering a gel composition as defined by clause 14. Preferred embodiments of the method are provided in the dependent claims and the description of the invention, which can optionally be combined with each other.

Further aspects of the invention relate to the use of the inventive powder composition for preparing a gel according to clause 16 and the use of the inventive powder composition or the inventive gel composition for feeding, vaccinating or health treatment of animals according to clause 17.

The inventive powder composition for use in feeding animals, especially poultry comprises or consists of,
- at least one gelling agent in an amount of 0,1 to 20 wt.% and preferably 0,5 to 15 wt.% based on the total amount of the powder composition, wherein the gelling agent is preferably a hydrocolloid;
- at least one carrier in an amount of 30 to 70 wt.% based on the total amount of the powder composition, wherein the at least one carrier is selected from the group consisting of saccharides, polyols, proteins or mixtures thereof, preferably saccharides; and
- a prebiotic additive, wherein the prebiotic additive is an aquatic photosynthesizing organism, preferably a microalgae, wherein the amount of the prebiotic additive is between 1 to 50 wt% based on the total amount of the powder composition.

The powder composition can be used for feeding all sorts of animals such as cattle, swine, poultry such as chicks or poults. The powder composition for use in feeding animals is particularly suitable for treating new born animals such as chicks.

The powder composition provides nutritional compounds being advantageous for the development of new born animals such as chicks. Besides, the powder composition can be used for preparing gels which can be easily administered and can easily be taken up by the animals.

The powder composition also has a good solubility in water. The powder composition also forms stable gels when mixed with water.

### Definitions

The term poultry denotes domesticated birds that are being kept for their eggs, meat, or feathers and includes, but is not limited to, chicken, turkeys, ducks, geese, and quail.

The term aquatic photosynthesizing organisms denotes organisms typically found in freshwater and marine systems, living in both the water column and sediment and being able to perform photosynthesis.

The term microalgae denotes microscopic algae. Microalgae are unicellular species existing individually or in chains or groups. Their sizes can range from a few micrometers (1 to 5 µm) to a few hundred micrometers. Microalgae do not have roots, stems or leaves. The term microalgae covers eukaryotic species such as Chlorella and in particular Chlorella vulgaris, as well as prokaryotic species like cyanobacteria, also called blue-green algae. A genus of cyanobacteria is Spirulina of what Arthrospira platensis and Arthrospira maxima are examples of.

In the context of this invention the term microalgae is used for microalgae capable of performing photosynthesis. The nutritional value of microalgae varies between species but protein (6-63%), carbohydrates (8-64%) and lipids (2-50%) are the major nutritional components found in microalgae. Microalgae may contain further compounds such as chlorophyll, carotenoids and phycocyanins.

Prebiotics according to this invention are compounds in animal feed or human food that induce the growth or activity of beneficial microorganisms such as bacteria and fungi. Prebiotic additives according to this invention are preferably prebiotic feed substances.

Probiotics denotes live microorganisms intended to provide health benefits when consumed, generally by improving or restoring the gut flora.

The term agent denotes a chemical or biological entity that can be administered to an animal. The agent can have a property that induces a physiological response in an animal such as poultry, cattle, horses, birds, pets, and the like as well as humans. An agent can comprise a single chemical or biological entity, combinations of chemical entities, combinations of biological entities, or combinations of chemical and biological entities. Non-limiting examples of agents include direct-fed microbials (DFMs), vaccines, competitive exclusion agents, antigens, peptides, immunomodulators, nutrients such as fat, carbohydrate, protein, one or more bacterial strain, combinations of bacterial strains, vitamins, minerals, prebiotic compounds, botanicals, non-nutritive feed additives, and antibiotics. DFMs are bacteria providing animals positive effects, including, but not limited to, reducing harmful pathogens and increasing performance. Performance measures include but are not limited to such parameters as average daily feed intake, average daily weight gain, total weight gain, European production factor, feed conversion, which includes both feed: gain and gain:feed, feed efficiency, mortality, and actual production costs.

The powder composition comprises at least one gelling agent. In the following the term gelling agent also denotes thickening agent. A gelling agent in the context of the invention is a compound being able to form a gel by dissolving in the liquid phase as a colloidal mixture and/or increasing the viscosity of a liquid it is added to by other mechanisms known to the person skilled in the art.

The powder composition comprises at least one gelling agent in an amount of 0,1 to 20 wt.%, preferably 0,5 to 15 wt.%, more preferably 5 to 15 wt.%, even more preferably 6 to 13 wt.%, and even more preferably 7 to 12 wt.% based on the total amount of the powder composition.

The gelling agent is preferably a hydrocolloid. Hydrocolloids are polysaccharides and proteins being able to form gels in water. Preferably the hydrocolloid is selected from the group of alginate, carrageenan, starch, gelatine, xanthan, cellulose, methylcellulose, carboxymethyl cellulose, glucomannan, konjac mannan, pectine, gum arabic, locust bean gum, guar gum, gum tragacanth and mixtures thereof, even more preferably carrageenan, alginate, xanthan, carboxymethyl cellulose, methylcellulose, konjac mannan, gum tragacanth and mixtures thereof, still more preferably xanthan, methylcellulose, konjac mannan, gum tragacanth and mixtures thereof and most preferably xanthan.

Preferably the at least one gelling agent is xanthan being present in an amount of 0,5 to 15 wt.% based on the total amount of the powder composition.

The powder composition comprises at least one carrier. The term carrier also denotes fillers. A carrier is a substance used to dissolve, dilute, disperse or otherwise physically modify a feed additive in order to facilitate its handling, application or use without altering its technological function and without exerting any technological effect itself.

The powder composition comprises at least one carrier in an amount of 30 to 70 wt.%, preferably 35 to 65 wt.%, and more preferably 40 to 60 wt.% based on the total amount of the powder composition.

The at least one carrier is selected from the group consisting of saccharides, polyols, proteins or mixtures thereof. Suitable proteins are whey permeate or sweet whey.

In a preferred embodiment the carrier is a saccharide, preferably a saccharide selected from the group of maltodextrin, inulin, dextrose, lactose or mixtures thereof, more preferably selected from maltodextrin, inulin and lactose, and even more preferably lactose.

Preferably the at least one carrier is present in an amount of 40 to 60 wt.% based on the total amount of the powder composition and selected from the group of maltodextrin, inulin, dextrose, lactose or mixtures thereof and more preferably is lactose.

The powder mixture comprises a prebiotic additive, wherein the prebiotic additive is an aquatic photosynthesizing organism. Preferably the prebiotic additive is a microalgae. More preferably the microalgae is selected from Chlorella, Spirulinales, Tetraselmis, Schizochytrium or mixtures thereof, even more preferably selected from Chlorella vulgaris, Arthrospira platensis, Arthrospira maxima, Tetraselmis chuii, Schizochytrium aggregatum or mixtures thereof, and most preferably the prebiotic additive is Arthrospira platensis.

According to a preferred embodiment the prebiotic additive is dried after the harvest of the aquatic photosynthesizing organisms. The drying is performed according to procedures known in the art.

In another embodiment the prebiotic additive is preferably a powder. A powder is a dry, bulk solid composed of a large number of fine particles that may flow freely when shaken or tilted.

Preferably the powder has a particle size of 40 to 200 mesh, more preferably of 60 to 160 mesh and even more preferably of 80 to 120 mesh.

The prebiotic additive is present in an amount between 1 to 50 wt%, preferably between 5 to 40 wt.%, and more preferably 10 to 30 wt.% based on the total amount of the powder composition.

In another embodiment the prebiotic additive is preferably a powder selected from the group of Chlorella vulgaris, Arthrospira platensis, Arthrospira maxima or mixtures thereof, preferably the prebiotic additive is Arthrospira platensis and the prebiotic additive is present in an amount between 5 to 40 wt., preferably 10 to 30 wt.% based on the total amount of the powder composition.

Preferably the prebiotic additive is green. More preferably the colour of the prebiotic additive in the CIELAB colour space (CIE L*a*b*) determined according to DIN EN ISO 11664-4 is, L* selected from a value between 20 and 60, a* selected from a value between - 20 and 20 and b* selected from a value between - 10 and 30, even more preferably L* selected from a value between 30 and 50, a* selected from a value between - 10 and 10 and b* selected from a value between 0 and 20 and most preferably L* selected from a value between 35 and 45, a* selected from a value between - 10 and 5 and b* selected from a value between 0 and 10.

In an embodiment the composition preferably comprises at least one further additive selected from the group of anti-caking agents, complexing agents, vitamins, oils, minerals, and electrolytes. These additives can be chosen from any known material suitable for animal foodstuffs.

Anti-caking agents are used to avoid clumping and caking in particular upon long-term storage of the powder composition. The anti-caking agent maintains the flowability of the powder composition.

In an embodiment of the powder composition preferably the at least one further additive is an anti-caking agent. Preferably the anti-caking agent is selected from the group consisting of silica, colloidal silica, silicic acid, diatomaceous earth, calcium silicate, calcium carbonate, magnesium carbonate and mixtures thereof, and more preferably the anti-caking agent is silicic acid.

Preferably the amount of anti-caking agent is 0,1 to 2 wt. % and more preferably 0,5 to 1,5 wt.% based on the total amount of the powder composition.

According to a preferred embodiment the anti-caking is silicic acid in an amount of 0,1 to 2 wt. % based on the total amount of the powder composition.

Complexing agents have the function to complex substances. Complexing agents are particularly relevant when the powder composition is used to form a gel.

Complexing agents can for example complex further additives and/or agents such as vaccines so the complexed additives and/or agents maintain their usability and effectiveness. Those complexing agents stabilize the further additives and/or agents.

Complexing agents can also be used to complex substances which might disturb the usability and effectiveness of the overall composition. Such disturbing substances can for example be chlorine present in the water used for the formation of the gel composition.

According to a preferred embodiment the complexing agent is an agent to complex a vaccine. Preferably the complexing agent is whey protein.

The complexing agent for complexing a vaccine is present in an amount of 0,5 to 8 wt% and preferably 1 to 6 wt.% based on the total amount of the powder composition.

Preferably the whey protein is present in an amount of 1 to 6 wt.% based on the total amount of the powder composition.

According to a preferred embodiment the complexing agent is an agent to complex impurities or other undesirable substances. Preferably this complexing agent is selected from ascorbate or thiosulfate and more preferably is sodium ascorbate.

Ascorbate or thiosulfate are present in the powder composition in an amount of 0,1 to 2 wt.% based on the total amount of the powder composition.

Preferably ascorbate is present in an amount of 0,1 to 2 wt.% based on the total amount of the powder composition. More preferably sodium ascorbate is present in an amount of 0,1 to 2 wt.% based on the total amount of the powder composition.

According to a preferred embodiment the powder composition comprises two complexing agents wherein the first complexing agent stabilizing a vaccine is present in an amount of 1 to 6 wt.% based on the total amount of the powder composition and the second complexing agent is ascorbate in amount of 0,1 to 2 wt.% based on the total amount of the powder composition.

Vitamins, minerals and electrolytes can be any known material suitable for animal feeding. Minerals also comprise trace elements.

In a further embodiment of the powder composition preferably the content of protein in the powder composition is at least 1 wt.%, preferably at least 3 wt.% and more preferably at least 8 wt.% based on the total amount of the powder composition.

In another embodiment of the powder composition preferably the content of protein is 50 wt.% or less and more preferably 40 wt.% or less based on the total amount of the powder composition.

In another embodiment of the composition preferably the content of protein is 2,5 to 50 wt.%, preferably 5 to 40 wt.% and more preferably 10 to 40 wt.% based on the total amount of the powder composition.

In another embodiment the powder composition comprises further agents. Preferably the agents are selected from the group of vaccines, medicaments, prebiotics, vitamins and/or probiotics.

According to another embodiment the powder composition comprises a second gelling agent. Preferably this second gelling agent is chosen from the group of alginate, carrageenan, starch, gelatine, xanthan, cellulose, carboxymethyl cellulose, pectine, gum arabic, locust bean gum, guar gum and mixtures thereof, even more preferably carrageenan, carboxymethyl cellulose and mixtures thereof. The second gelling agent is different from the first, at least one gelling agent.

The powder composition comprises the second gelling agent in an amount of 0,1 to 20 wt.%, preferably 0,5 to 15 wt.%, more preferably 2 to 10 wt.%, even more preferably 3 to 9 wt.%, and even more preferably 4 to 8 wt.% based on the total amount of the powder composition.

In another embodiment of the powder composition the colour of the overall composition is green. Preferably the colour of the of the overall composition in the CIELAB colour space (CIE L*a*b*) determined according to DIN EN ISO 11664-4 is, L* selected from a value between 30 and 80, a* selected from a value between - 20 and 20 and b* selected from a value between - 10 and 30, even more preferably L* selected from a value between 40 and 70, a* selected from a value between - 10 and 10 and b* selected from a value between 0 and 20 and most preferably L* selected from a value between 50 and 70, a* selected from a value between -10 and 5 and b* selected from a value between 0 and 10.

Powder compositions with above colour are recognized in particular by chicken especially well. By that the intake of the powder composition is increased leading to a more efficient feeding of the animals in particular chickens and chicks.

In a preferred embodiment the powder composition comprises or consists of at least one gelling agent in an amount of 0,5 to 15 wt.% based on the total amount of the powder composition, wherein the gelling agent is a polysaccharide, preferably xanthan;
at least one carrier in an amount of 35 to 65 wt.% based on the total amount of the powder composition, wherein the at least one carrier is a saccharide selected from the group of maltodextrin, inulin, dextrose, lactose or mixtures thereof, preferably is lactose; and
a prebiotic additive, selected from the group of Spirulinales and preferably is Arthrospira platensis, wherein the amount of the prebiotic additive is between 5 to 40 wt.% based on the total amount of the powder composition.

According to a further preferred embodiment the powder composition comprises or consists of at least one gelling agent in an amount of 0,5 to 15 wt.% based on the total amount of the powder composition, wherein the gelling agent is a polysaccharide, preferably xanthan;
at least one carrier in an amount of 35 to 65 wt.% based on the total amount of the powder composition, wherein the at least one carrier is a saccharide selected from the group of maltodextrin, inulin, dextrose, lactose or mixtures thereof, preferably is lactose;
a prebiotic additive, selected from the group of Spirulinales and is preferably Arthrospira platensis, wherein the amount of the prebiotic additive is between 5 to 40 wt.% based on the total amount of the powder composition; and
an anti-caking agent wherein the anti-caking agent is silicic acid in an amount of 0,1 to 2 wt. % based on the total amount of the powder composition.

According to another preferred embodiment the powder composition comprises or consists of at least one gelling agent in an amount of 0,5 to 15 wt.% based on the total amount of the powder composition, wherein the gelling agent is xanthan;
at least one carrier in an amount of 35 to 65 wt.% based on the total amount of the powder composition, wherein the at least one carrier is a saccharide selected from the group of maltodextrin, inulin, dextrose, lactose or mixtures thereof, preferably is selected from maltodextrin or lactose;
a prebiotic additive wherein the prebiotic additive is Arthrospira platensis, wherein the amount of the prebiotic additive is between 5 to 40 wt.% based on the total amount of the powder composition; and
an anti-caking agent wherein the anti-caking agent is silicic acid in an amount of 0,1 to 2 wt. % based on the total amount of the powder composition.

According to a further preferred embodiment the powder composition comprises or consists of at least one gelling agent in an amount of 0,5 to 15 wt.% based on the total amount of the powder composition, wherein the gelling agent is a polysaccharide, preferably is xanthan;
at least one carrier in an amount of 35 to 65 wt.% based on the total amount of the powder composition, wherein the at least one carrier is a saccharide selected from the group of maltodextrin, inulin, dextrose, lactose or mixtures thereof, preferably lactose;
a prebiotic additive, selected from the group of Spirulinales and preferably is Arthrospira platensis, wherein the amount of the prebiotic additive is between 5 to 40 wt.% based on the total amount of the powder composition;
an anti-caking agent wherein the anti-caking agent is silicic acid in an amount of 0,1 to 2 wt. % based on the total amount of the powder composition; and
a complexing agent, wherein the complexing agent is ascorbate in an amount of 0,1 to 2 wt.% based on the total amount of the powder composition.

According to a further preferred embodiment the powder composition comprises or consists of at least one gelling agent in an amount of 0,5 to 15 wt.% based on the total amount of the powder composition, wherein the gelling agent is a polysaccharide, preferably is xanthan;
at least one carrier in an amount of 35 to 65 wt.% based on the total amount of the powder composition, wherein the at least one carrier is a saccharide selected from the group of maltodextrin, inulin, dextrose, lactose or mixtures thereof, preferably lactose;
a prebiotic additive, wherein the prebiotic additive is Chlorella vulgaris, wherein the amount of the prebiotic additive is between 5 to 40 wt.% based on the total amount of the powder composition;
an anti-caking agent wherein the anti-caking agent is silicic acid in an amount of 0,1 to 2 wt. % based on the total amount of the powder composition; and
a complexing agent, wherein the complexing agent is ascorbate in an amount of 0,1 to 2 wt.% based on the total amount of the powder composition.

In a preferred embodiment the powder composition comprises or consists of at least one gelling agent in an amount of 0,5 to 15 wt.% based on the total amount of the powder composition, wherein the gelling agent is a polysaccharide, preferably xanthan;
at least one carrier in an amount of 35 to 65 wt.% based on the total amount of the powder composition, wherein the at least one carrier is a saccharide selected from the group of maltodextrin, inulin, dextrose, lactose or mixtures thereof, preferably is lactose;
a prebiotic additive, selected from the group of Spirulinales and preferably is Arthrospira platensis, wherein the amount of the prebiotic additive is between 5 to 40 wt.% based on the total amount of the powder; and
wherein composition wherein the colour of the prebiotic additive in the CIELAB colour space (CIE L*a*b*) determined according to DIN EN ISO 11664-4 is, L* selected from a value between 20 and 60, a* selected from a value between - 20 and 20 and b* selected from a value between - 10 and 30, and preferably is L* selected from a value between 35 and 45, a* selected from a value between - 10 and 5 and b* selected from a value between 0 and 10.

In a preferred embodiment the powder composition comprises or consists of at least one gelling agent in an amount of 0,5 to 15 wt.% based on the total amount of the powder composition, wherein the gelling agent is a polysaccharide, preferably xanthan;
at least one carrier in an amount of 35 to 65 wt.% based on the total amount of the powder composition, wherein the at least one carrier is a saccharide selected from the group of maltodextrin, inulin, dextrose, lactose or mixtures thereof, preferably is lactose; and
a prebiotic additive, selected from the group of Spirulinales and preferably is Arthrospira platensis, wherein the amount of the prebiotic additive is between 5 to 40 wt.% based on the total amount of the powder composition; and
wherein the colour of the overall composition in the CIELAB colour space (CIE L*a*b*) determined according to DIN EN ISO 11664-4 is, L* selected from a value between 30 and 80, a* selected from a value between - 20 and 20 and b* selected from a value between - 10 and 30, and preferably L* selected from a value between 50 and 70, a* selected from a value between - 10 and 5 and b* selected from a value between 0 and 10.

Another aspect of the invention relates to a gel composition for use in animal feeding comprising or consisting of
- a powder composition according to clauses 1 to 7 and
- water,
wherein the powder composition is suspended in the water to form the gel composition.

Specific embodiments provided with respect to the powder composition apply also to the gel composition. Preferred embodiments of the powder composition are also preferred embodiments of the gel composition.

The inventive gel composition for use in animal feeding comprises or consists of the inventive powder composition and water, wherein the powder composition is suspended in the water to form the gel composition.

The inventive gel composition stays on the fur or feather of the animals. Extensive wetting is avoided which can lead to a decrease in body temperature. In particular for new born animals, especially chicks this is of great importance as a loss of body temperature can lead to death easily. The gel compositions provide the animals, especially the chicks, with nutrition and important substances relevant for their health and physical well-being. Furthermore, the inventive gel composition may have a colour that is easily recognized by poultry in particular chicks.

Due to the nature of a gel, additional substances that may be added are more evenly suspended in the gel composition. Sedimentation does not form as quickly as in water, thus minimizing the risk of uneven distribution of additives and/or agents such as vaccines.

The gel composition is formed by suspending the powder composition in water, whereby a gel is formed.

In an embodiment of the gel composition preferably the ratio of the powder composition and water is between 1:1 and 1:100 by weight, preferably between 1:1 and 1:75 by weight, more preferably 1:3 to 1:50 by weight, even more preferably 1:10 to 1:40 by weight and still even more preferably 1:20 to 1:30 by weight. Gels are obtained which can be administered to animals in particular chicks without soaking of the feathers. By applying above ratios preferred consistencies of the gel are obtained.

Sedimentation of additives, agents and other substances added to the gel composition is decreased. By that the risk of overdosing certain substances is reduced due to uneven distribution of substances like additives, agents and the like present in the gel composition.

According to another embodiment the gel composition has a viscosity of 10 - 500 mPa ^{•} s, preferably 10 - 250 mPa ^{•} s, more preferably of 30 - 200 mPa ^{•} s, even more preferably of 50 - 150 mPa ^{•} s, and most preferably of 80 - 120 mPa ' s. Gels with such viscosities have advantageous properties for administration to the animals. In addition to that, the droplet formation is enhanced further decreasing the wetting of the fur or feathers of the animals.

In a preferred embodiment the ratio of the powder composition and water is between 1:3 to 1:50 by weight and the viscosity of the gel composition is 50 - 150 mPa ^{•} s.

In another preferred embodiment the amount at least one gelling agent in the powder composition is 0,5 to 15 wt.% based on the total amount of the powder composition, and the viscosity of the gel composition is 50 - 150 mPa ' s.

In another preferred embodiment the amount of the at least one gelling agent in the gel composition is 0,01 to 0,75 wt.%, preferably 0,05 to 0,5 wt.% and more preferably 0,1 to 0,5 wt.% based on the total amount of the gel composition, and the viscosity of the gel composition is 50 - 150 mPa ^{•} s.

In a further embodiment of the gel composition the gel composition comprises at least one further agent being selected from the groups of vaccines, provitamins, vitamins, betaine, prebiotics, and/or probiotics.

Betaine is preferably selected from the group of betaine hydrochloride or betaine anhydrate.

The prebiotic is preferably mannan-oligosaccharide.

Probiotics are preferably selected from the group of lactic acid bacteria, spore-forming bacteria or mixtures thereof. More preferably the probiotics are selected from the group of Enterococcus faecium, Bacillus spp. or mixtures thereof and even more preferably selected from the group of Enterococcus faecium, Bacillus subtilis, Bacillus licheniformis or mixtures thereof.

In a further embodiment of the gel composition preferably the amount of the prebiotic additive is between 0,1 to 15 wt%, preferably between 0,25 to 12,5 wt.%, more preferably 0,4 to 5 wt.% and even more preferably 0,5 to 2 wt.% based on the total amount of the gel composition.

In a preferred embodiment of the gel composition the colour of the gel composition is green.

The green colour of the gel composition enhances the take up of the gel composition by the animals in particular the chicks. When the gel is on the feathers it stays on the feathers and does not soak them. In addition to that, chicks are able to detect the composition more easily and start to pick the composition from the feathers as the specific colour perception of chicks and their ability to recognize green colour is especially well.

In an embodiment the gel composition has at least two absorption maxima in the range of 600 to 700 nm. Preferably the first absorption maximum is in the range of 600 to 640 nm and the second absorption maximum is in the range of 665 to 690 nm.

In a further embodiment the gel composition has at least three absorption maxima in the range of 400 to 700 nm. Preferably the first absorption maximum is in the range of 600 to 640 nm, the second absorption maximum is in the range of 665 to 690 nm and the third absorption maximum is in the range of 400 to 450 nm.

The absorption of the gel can be determined via photometric measurements using a PerkinElmer LAMBDA 25 apparatus applying methods known in art. Gels are diluted in a ratio of 1:100.

In another aspect the invention provides a kit for treating animals the kit comprising or consisting of the inventive powder composition in a first container and an agent, preferably a vaccine, probiotic or medicament, in another container.

Specific embodiments provided with respect to the powder composition and the gel composition apply also to the kit for treating animals. Preferred embodiments of the powder composition and gel composition are also preferred embodiments of the kit for treating animals.

A further aspect of the present invention relates to a method for administering a gel composition comprising the steps of
a) preparing a gel composition according to clauses 8 to 13; and
b) administering the gel composition to animals, preferably poultry, and more preferably chicks.

Specific embodiments provided with respect to the powder composition and the gel composition apply also to the method for administering a gel composition. Preferred embodiments of the powder composition and gel composition are also preferred embodiments of the method for administering the gel composition.

According to an embodiment the method for administering a gel composition comprises or consists of the steps of
a) preparing a gel composition; and
b) administering the gel composition to animals, preferably poultry, and more preferably chicks.

The gel is preferably administered to poultry and more preferably to chicks. The gel composition stays on the fur or feathers of the animals so that it can be detected and taken up by the animals.

According to an embodiment of the gel composition, in step b) the gel composition is sprayed, sprinkled or dripped on the animals, preferably poultry, and more preferably chicks.

Another aspect of the invention relates to the use of the powder composition according to the invention for preparing a gel.

A further aspect of the invention relates to the use of a powder composition according to the invention or a gel composition according to the invention for feeding, vaccinating or health treatment of animals, preferably poultry and more preferably chicks.

### Experimental Section

In the following the invention is described by, but not limited to, examples.

### Viscosity Measurement

A rotation-rheometer PCE-RVI 2 V1R was used for the measurements. The viscosity was determined as follows: A beaker glass containing a gel composition was placed under the rheometer using a spindle type R3 and the speed of rheometer was set to 200 rpm. It was measured at a capacity of at least 10 %.

### Materials & apparatus

Lactose and whey powder (WPC 35) were used in feed grade. Xanthan (E415), silicic acid (E551a) and sodium ascorbate (E301) were used in feed grade. Algomed^{®} Chlorella powder, a powder of the microalgae *Chlorella vulgaris,* was purchased from Roquette Klötze GmbH & Co KG (Klötze, Germany).

The blender used for the experiments was a Siemens MQ 67110.

### Examples

In the following the preparation of inventive gel compositions is described. For the preparation of the inventive gels inventive powder compositions were used. The compositions of the powder compositions used for the preparation of inventive gel compositions, Inventive Examples 1 to 3 (IE1 to IE 3), are shown in Table 1.

**Table 1**

| | **IE1** | **IE2** | **IE3** |
|---|---|---|---|
| | wt.% | wt.% | wt.% |
| | | | |
| lactose | 66,25 | 57,75 | 51,75 |
| whey powder (WPC 35) | 6,25 | 6,25 | 6,25 |
| Algomed^{®} Chlorella powder | 25 | 25 | 25 |
| silicic acid (E551a) | 1 | 1 | 1 |
| xanthan (E415) | 0,5 | 9 | 15 |
| sodium ascorbate (E301) | 1 | 1 | 1 |

### Preparation of powder compositions

The powder compositions were prepared by mixing and grinding lactose, whey powder, Algomed^{®} Chlorella powder, silicic acid, sodium ascorbate and xanthan at room temperature (25°C) until an even blend was obtained and no clumps were detectable.

### Preparation of gel compositions

The gel compositions were prepared by using the respective amounts of powder composition and water as listed in Table 2. The gel compositions were prepared by submerging a blender into a beaker glass containing the water. During constant mixing the respective powder composition was slowly poured into the water. The mixture was blended for two minutes after addition of the powder composition. An even gel composition was obtained.

**Table 2**

| | **powder composition (g)** | **water (g)** | **weight ratio powder to water** |
|---|---|---|---|
| | | | |
| **IE1** | 100 | 300 | 1:3 |
| **IE2** | 100 | 2500 | 1:25 |
| **IE3** | 100 | 5000 | 1:50 |

### Measurement of the viscosity

100 ml of the gel compositions of IE1 to IE3, respectively, were filled into a 100 ml beaker glass for measuring the viscosity of the gel compositions according to the method described under measurement methods.

The results of the viscosity measurements are shown in Table 3.

**Table 3**

| | **temperature (°C)** | **capacity (%)** | **viscosity (mPa^{•}s)** |
|---|---|---|---|
| **IE1** | 28,4 | 24 | 120 |
| **IE2** | 26,7 | 22 | 110 |
| **IE3** | 27,1 | 17,5 | 80 |

The inventive powder compositions showed good solubility in water. It was possible to apply them in different powder to water ratios, thereby still obtaining suitable gel composition.

The obtained gel compositions were soft and flowable. They had a viscosity in the range of 80 to 120 mPa ^{•} s. The gel compositions are of green colour and can easily be detected by chicks and due to their viscosity the gel compositions stay on the feather for a sufficient amount of time to be picked by chicks. Wetting of the feathers leading to decreased body temperature is avoided. The mortality rate can be decreased.

The viscosity of the inventive gel composition allows its easy administration via spraying or dripping. It avoids blocking of the administration devices. Hence, it can be applied on hatchlings in the hatchery as well as in a barn.

Although specific embodiments have been illustrated and described herein, it will be appreciated by those of ordinary skill in the art that any arrangement that is calculated to achieve the same purpose may be substituted for the specific embodiments shown. This application is intended to cover any adaptations or variations that operate according to the principles of the invention as described. Therefore, it is intended that this invention be limited only by the claims and the equivalents thereof.

In the following clauses, preferred embodiments of the invention are described:
1. A powder composition for use in feeding animals, especially poultry comprising
   - at least one gelling agent in an amount of 0,1 to 20 wt.% and preferably 0,5 to 15 wt.% based on the total amount of the powder composition, wherein the gelling agent is preferably a hydrocolloid;
   - at least one carrier in an amount of 30 to 70 wt.% based on the total amount of the powder composition,
      wherein the at least one carrier is selected from the group consisting of saccharides, polyols, proteins or mixtures thereof, preferably saccharides; and
   - a prebiotic additive, wherein the prebiotic additive is an aquatic photosynthesizing organism, preferably a microalgae,
      wherein the amount of the prebiotic additive is between 1 to 50 wt.% based on the total amount of the powder composition.
2. The powder composition according to clause 1 comprising at least one further additive selected from the group of anti-caking agents, complexing agents, vitamins, oils, minerals, and electrolytes.
3. The powder composition according to clause 2 wherein the at least one further additive is an anti-caking agent, preferably silicic acid, wherein the amount of anti-caking agent is 0,1 to 2 wt. % based on the total amount of the powder composition.
4. The powder composition according to clause 2,
   wherein the further additive is a complexing agent, and
   wherein the complexing agent is whey protein in an amount of 0,5 to 8 wt.%, preferably 1 to 6 wt.% based on the total amount of the powder composition, or
   wherein the further additive is selected from ascorbate or thiosulfate, preferably sodium ascorbate, in an amount of 0,1 to 2 wt.% based on the total amount of the powder composition.
5. The composition according to any of the preceding clauses, wherein the content of protein in the powder composition is at least 1 wt.%, preferably at least 3 wt.% and more preferably at least 8 wt.% based on the total amount of the powder composition.
6. The powder composition according to any of the preceding clauses wherein the colour of the overall composition is green, preferably the colour of the overall composition in the CIELAB colour space (CIE L*a*b*) determined according to DIN EN ISO 11664-4 is, L* selected from a value between 30 and 80, a* selected from a value between - 20 and 20 and b* selected from a value between - 10 and 30, and more preferably L* selected from a value between 50 and 70, a* selected from a value between - 10 and 5 and b* selected from a value between 0 and 10.
7. The powder composition according to any of the preceding clauses wherein the prebiotic additive is selected from the group of Spirulinales, and preferably is Arthrospira platensis, and wherein the amount of the prebiotic additive is between 5 to 40 wt.%, and preferably 10 to 30 wt.% based on the total amount of the powder composition.
8. A gel composition for use in animal feeding comprising
   - a powder composition according to clauses 1 to 7 and
   - water,
   wherein the powder composition is suspended in the water to form the gel composition.
9. The gel composition according to clause 8 wherein the ratio of the powder composition and water is between 1:1 and 1:50 by weight, preferably 1:3 to 1:50 by weight, more preferably 1:10 to 1:40 by weight and even more preferably 1:20 to 1:30 by weight.
10. The gel composition according to clauses 8 or 9 wherein the gel composition has a viscosity of 10 - 250 mPa ^{•} s, preferably of 30 - 200 mPa ^{•} s, more preferably of 50 - 150 mPa ^{•} s, and most preferably of 80 - 120 mPa ^{•} s.
11. The gel composition according to any of the clauses 8 to 10 wherein the gel composition comprises at least one further agent being selected from the groups of vaccines, provitamins, vitamins, betaine, prebiotics, and/or probiotics.
12. The gel composition according to any of the clauses 8 to 11, wherein the amount of the prebiotic additive is between 0,1 to 15 wt.%, preferably between 0,25 to 12,5 wt.%, more preferably 0,4 to 5 wt.% and even more preferably 0,5 to 2 wt.% based on the total amount of the gel composition.
13. The gel composition according to any of the clauses 8 to 12, wherein the colour of the gel composition is green.
14. Method for administering a gel composition comprising the steps of a) preparing a gel composition according to clauses 8 to 13; and b) administering the gel composition to animals, preferably poultry, and more preferably chicks.
15. The method for administering a gel composition according to clause 14 wherein in step b) the gel composition is sprayed, sprinkled or dripped on the animals, preferably poultry, and more preferably chicks.
16. Use of the powder composition according to clauses 1 to 7 for preparing a gel.
17. Use of a powder composition according to clauses 1 to 7 or a gel composition according to clauses 8 to 13 for feeding, vaccinating or health treatment of animals, preferably poultry and more preferably chicks.

## Claims

1. A powder composition for use in feeding animals, especially poultry comprising
- at least one gelling agent in an amount of 0,1 to 20 wt.% and preferably 0,5 to 15 wt.% based on the total amount of the powder composition;
- at least one carrier in an amount of 30 to 70 wt.% based on the total amount of the powder composition,
wherein the at least one carrier is selected from the group consisting of saccharides; and
- a prebiotic additive, wherein the prebiotic additive is an aquatic photosynthesizing organism,
wherein the amount of the prebiotic additive is between 1 to 50 wt.% based on the total amount of the powder composition, and wherein the prebiotic additive is green;
wherein the content of protein in the powder composition is at least 3 wt.%; and
wherein the colour of the overall composition is green.

2. The powder composition according to claim 1 comprising at least one further additive selected from the group of anti-caking agents, vitamins, oils, minerals, and electrolytes.

3. The powder composition according to claim 2 wherein the at least one further additive is an anti-caking agent, preferably silicic acid, wherein the amount of anti-caking agent is 0,1 to 2 wt. % based on the total amount of the powder composition.

4. The powder composition according to claim 2,
wherein the further additive is selected from ascorbate or thiosulfate, preferably sodium ascorbate, in an amount of 0,1 to 2 wt.% based on the total amount of the powder composition.

5. The composition according to any of the preceding claims, wherein the content of protein in the powder composition is at least 8 wt.% based on the total amount of the powder composition; and/or
wherein the content of protein in the powder composition is 50 wt.% or less and preferably 40 wt.% or less based on the total amount of the powder composition; and/or
wherein the content of protein in the powder composition is 5 to 40 wt.% and preferably 10 to 40 wt.% based on the total amount of the powder composition; and/or
wherein the amount of the prebiotic additive is between 5 to 40 wt.%, and more preferably 10 to 30 wt.% based on the total amount of the powder composition.

6. The powder composition according to any of the preceding claims wherein the colour of the overall composition in the CIELAB colour space (CIE L*a*b*) determined according to DIN EN ISO 11664-4 is, L* selected from a value between 30 and 80, a* selected from a value between - 20 and 20 and b* selected from a value between - 10 and 30, and more preferably L* selected from a value between 50 and 70, a* selected from a value between - 10 and 5 and b* selected from a value between 0 and 10.

7. The powder composition according to any of the preceding claims wherein the prebiotic additive is selected from the group of Spirulinales, and preferably is Arthrospira platensis, and wherein the amount of the prebiotic additive is between 5 to 40 wt.%, and preferably 10 to 30 wt.% based on the total amount of the powder composition.

8. The powder composition according to any of the preceding claims wherein the prebiotic additive is a powder; and/or wherein the powder composition comprises further agents selected from the group of vaccines, medicaments, prebiotics, vitamins and/or probiotics.

9. The powder composition according to claim 8 wherein the prebiotic additive powder has a particle size of 40 to 200 mesh.

10. The powder composition according to any of the preceding claims wherein the prebiotic additive is dried after the harvest of the aquatic photosynthesizing organisms.

11. A gel composition for use in animal feeding comprising
- a powder composition according to claims 1 to 10 and
- water,
wherein the powder composition is suspended in the water to form the gel composition.

12. Use of the powder composition according to claims 1 to 10 for preparing a gel.

13. Use of a powder composition according to claims 1 to 10 or a gel composition according to claim 11 for feeding of animals, preferably poultry.

14. A powder composition according to claims 1 to 10 or a gel composition according to claim 11 for use in health treatment of animals, preferably poultry.

15. A kit for treating animals the kit comprising the powder composition according to claims 1 to 10 in a first container and a vaccine, probiotic or medicament, in another container.
